# NEW EUROPEAN PATENT SPECIFICATION

(11) **EP 1 117 736 B2**
(45) Date of publication and mention of the opposition decision: **13.08.2008**
(45) Mention of the grant of the patent: 14.07.2004
(21) Application number: 99939129.5
(22) Date of filing: 11.08.1999
(51) Int. Cl.: C08L 3/08, C08L 3/18, C09D 103/08, C08L 3/10, C09D 103/10, A61K 9/48, A61K 9/50

(54) **MODIFIED STARCH FILM COMPOSITIONS**
FILMBILDENDE ZUSAMMENSETZUNGEN AUS MODIFIZIERTER STÄRKE
COMPOSITIONS DE FILM D'AMIDON MODIFIE

(30) Priority: 30.09.1998 FR 9812246; 29.01.1999 US 240504
(43) Date of publication of application: 25.07.2001
(73) Proprietor: Warner-Lambert Company LLC, New York, NY 10017 (US)
(72) Inventor: SCOTT, Robert Anthony, B-9100 Sint-Niklaas (BE); CADE, Dominique, F-68000 Colmar (FR); HE, Xiongwei, F-68280 Andolsheim (FR)
(74) Representative: Dekker, Henrike Cornelie Christine
(86) International application number: PCT/US1999/018139
(87) International publication number: WO 2000/018835

(56) References cited:
- EP-A- 0 547 551
- EP-A- 0 606 486
- EP-B- 1 105 107
- WO-A-00/10538
- WO-A-95/00123
- US-A- 3 378 546
- US-A- 4 026 986
- US-A- 4 738 724
- US-A- 4 804 542
- US-A- 5 112 445
- US-A- 5 224 989
- US-A- 5 264 223
- US-A- 5 342 626
- US-A- 5 614 217
- US-A- 5 756 123
- DATABASE WPI Week 199307 Derwent Publications Ltd., London, GB; AN 1993-055171 XP002122906 "Soft capsule agent having controlled adhesivity during packaging etc. - has blended food fibre e.g. agar, carrageenan etc. in capsule film" & JP 05 004914 A (ASAHI KASEI KOGYO KK ET AL.), 14 January 1993 (1993-01-14)
- Starch/Staerke 47 (1995), Nr. 10, S. 378-384

## Description

The invention concerns aqueous compositions from hydroxpropylated starch (HPS) and hydroxyethylated (HES) for the manufacture of hard capsules. The hard capsules obtained by the present invention are similar to hard gelatine capsules (HGC).

A second embodiment of the invention is the use of the modified starch compositions for the manufacturing of hard capsules by conventional dip moulding process as normally used in the production of conventional hard gelatine capsules.

For the industrial manufacture of pharmaceutical capsules gelatine is most preferred for its gelling, film forming and surface active properties. The manufacture of hard gelatine capsules by dip moulding process exploits fully its gelling and film forming abilities. Such capsules are manufactured by dipping mould pins into a hot solution of gelatine, removing the pins from the gelatine solution, allowing the gelatine solution attached on pins to set by cooling, drying and stripping the so-formed shells from the pins. The setting of the solution on the mould pins after dipping is the critical step to obtain a uniform thickness of the capsule shell.

Attempts have been made to manufacture capsules with materials other than gelatine, notably with modified cellulose. Successful industrial examples are the capsules made of hydroxypropyl methylcellulose (HPMC). The HPMC capsules show several advantages over HGC. However, the raw material HPMC is significantly more expensive than gelatine.

Starch is another abundant natural polysaccharide which is renewable, biodegradable and of low cost. Because of the limited film forming ability and poor mechanical properties, the success in this field is more limited. A unique industrial example (US 4,738,724) are starch capsules produced by injection moulding, but such capsules have a much higher shell thickness and a different shape which requires specific filling and closing equipment.

US 4,026,986 describes the manufacture of HPS capsules by dip moulding process. However, due to the absence of setting ability of HPS solution, the dipping time is long (20 seconds), and therefore it did not result in commercial process.

Surprisingly, we found that the addition of a very small amount of a setting system, consisting of hydrocolloids, preferably polysaccharides, confers to HPS OR HES solution an appropriate setting ability with the result that hard HPS OR HES capsules can be manufactured by the dip moulding process of hard gelatine capsules under conventional process conditions.

The aim of the invention is therefore the provision of aqueous compositions based on HPS or HES for the manufacture of hard capsules and wherein the HPS or HES compositions have in aqueous solution a sufficient setting ability.

The first object of the invention is aqueous compositions as defined in claim 1 based on HPS or HES in an amount of 20% to 40% by weight of the aqueous solution to improve and adjust the mechanical properties of films for various applications.

We found that the addition of a plasticizer in the formulation can improve dramatically the HPS OR HES film flexibility. The plasticizer or mixture of plasticizers is selected from polyethylene glycol, glycerol, sorbitol, sucrose, corn syrup, fructose, dioctyl-sodium sulfosuccinate, triethyl citrate, tributyl citrate, 1,2-propylenglycol, mono-, di- or triacetates of glycerol, or natural gums. Preferred are glycerol, polyethylene glycol, propylene glycol, citrates and their combinations. The amount of plasticizer depends on the final application. For hard film formulations, such as for hard capsules, the plasticizer is contained in an amount of 0 to 20%, preferably 10-20%. Even though it is not part of the invention, it is noted that a higher content, 20-30%, is preferred for soft film formulations, such as for soft capsules.

We found also that it is possible to further improve the film mechanical properties, by combining the HPS or HES with other hydrosoluble polymers or polysaccharides. The preferable examples are pectin, alginates, polyvinyl alcohol and high molecular weight polyethylene glycol.

The second object of the present invention is the achievement of an adequate setting ability of the HPS OR HES solution for process purpose.

The addition of a setting system, consisting of cations in an amount of 0.01 to 1% by weight of the aqueous solution and a hydrocolloid or mixtures of hydrocolloids in an amount of 0.03 to 1% by weight of the aqueous solution, to HPS OR HES solutions enables the adaptation of specific and desired gelling properties for a selected process (film forming or dip moulding such as the production of hard HPS OR HES capsules by a conventional dipping process). For the production of hard capsules by dip moulding process, it is extremely important that the film forming HPS OR HES solution remaining on the mould pins after dipping is prohibited from flowing down the pins. Otherwise the obtained film will not have the desired uniform thickness.

with the compositions of the present invention we can produce hard HPS OR HES capsules with the same equipment and in the same range of process conditions as used for the production of conventional hard gelatine capsules. Furthermore capsules produced from compositions of the instant invention have the same dimensional specifications and allow the use of the existing filling machinery and do not require specific and new equipment for the filling process.

The HPS OR HES concentration in the dipping solution is in a range of 20 to 40% by weight.

The setting system consists of a hydrocolloid or mixtures of hydrocolloids in the amounts specified above and contain in addition cations and/or sequestering agents.

Suitable hydrocolloids or mixtures producing synergistic properties may be selected from natural seaweeds, natural seed gums, natural plant exudates, natural fruit extracts, biosynthetic gums, biosynthetic processed starch or cellulosic materials, preferred are polysaccharides.

The preferred polysaccharides are alginates, agar gum, guar gum, locust bean gum (carob), carrageenan, tara gum, gum arabic, ghatti gum, Khaya grandifolia gum, tragacanth gum, karaya gum, pectin, arabian (araban), xanthan, gellan, starch, Konjac mannan, galactomannan, funoran, and other exocellular polysaccharides. Preferred are exocellular polysaccharides.

The preferred exocellular polysaccharides are xanthan, acetan, gellan, welan, rhamsan, furcelleran, succinoglycan, scleroglycan, schizophyllan, tamarind gum, curdlan, pullulan, and dextran.

The preferred hydrocolloids are kappa-carrageenan or gellan gum or combinations like xanthan with locust bean gum or xanthan with konjac mannan.

Among the setting systems mentioned above, the systems of kappa-carrageenan with cation and gellan gum with cation are specifically preferred. They produce high gel strength at low concentrations and have excellent compatibility with HPS.

The amount of the hydrocolloid is in the range of 0.03 to 1.0% by weight in the aqueous HPS OR HES solution.

The cations are preferably selected from K⁺, Na⁺, Li⁺, NH₄⁺, Ca⁺⁺ or Mg⁺⁺, for kappa-carrageenan is preferred K⁺, NH₄⁺ or Ca⁺⁺. The amount of cations is 0.01 to 1% by weight in the aqueous HPS OR HES solution.

The preferred sequestering agents are ethylenediaminetetraacetic acid, acetic acid, boric acid, citric acid, edetic acid, gluconic acid, lactic acid, phosphoric acid, tartaric acid or salts thereof, methaphosphates, dihydroxyethylglycine, lecithin or beta cyclodextrin and combinations thereof. Especially preferred is ethylenediaminetetraacetic acid or salts thereof or citric acid or salts thereof. The amount is preferably less than 3%, especially 0.01 to 1% by weight of the dipping solution.

The inventive HPS OR HES compositions may contain in a further aspect additional pharmaceutically or food acceptable colouring agents in the range of from 0 to 10% based upon the weight of the film. The colouring agents may be selected from azo-, quinophthalone-, triphenylmethane-, xanthene- or indigoid dyes, iron oxides or hydroxides, titanium dioxide or natural dyes or mixtures thereof. Examples are patent blue V, acid brilliant green BS, red 2G, azorubine, ponceau 4R, amaranth, D+C red 33, D+C red 22, D+C red 26, D+C red 28, D+C yellow 10, yellow 2 G, FD+C yellow 5, FD+C yellow 6, FD+C red 3, FD+C red 40, FD+C blue 1, FD+C blue 2, FD+C green 3, brilliant black BN, carbon black, iron oxide black, iron oxide red, iron oxide yellow, titanium dioxide, riboflavin, carotenes, anthocyanines, turmeric, cochineal extract, clorophyllin, canthaxanthin, caramel, or betanin.

The HPS OR HES capsules of the invention may be coated with a suitable coating agent like cellulose acetate phthalate, polyvinyl acetate phtbalate, methacrylic acid gelatines, hypromellose phthalate, hydroxypropylmethyl cellulose phthalate, hydroxyalkyl methyl cellulose phthalates or mixtures thereof to provide e.g. enteric properties.

The HPS OR HES capsules of the invention may be used for the production of containers for providing unit dosage forms for example for agrochemicals, seeds, herbs, foodstuffs, dyestuffs, pharmaceuticals, flavouring agents and the like.

The HPS OR HES capsules of the invention may be used where a release of filled product must occur at low temperature, such as at room temperature, which is not achievable with gelatine capsules.

The following examples and tests demonstrate the HPS OR HES capsule production and properties:

### Example 1: Production of HPS capsules with 15% plasticizer

1.5 kg of HPS powder is mixed with 25 g of kappa-carrageenan. To 3.21 kg of deionised water under stirring is added 0.5 g of potassium acetate (0.01% by weight in the solution) and 265 g of glycerol (5.3% in solution and 15% in capsule), followed by addition of the above mixture (30% of HPS and 0.5% of carrageenan in the solution). After the HPS is well dispersed, the dispersion is heated up to 90°C under slow stirring, then held under strong stirring for 10 minutes to assure a good solubilisation of the components.

The HPS solution thus prepared is defoamed under slow stirring and then poured into a dipping dish of a pilot machine of conventional hard gelatine capsule production equipment. While keeping the dipping HPS solution at 60°C. natural transparent hard HPS capsules of size 0 were produced according to the conventional process with the same dimensional specifications to the conventional hard gelatine capsules.

Disintegration test results (according to USP XXIII 1995-<701> Disintegration):

| | |
|---|---|
| First leak time | 21 seconds |
| Total disintegration time | 263 seconds. |

### Example 2: Production of HPS capsules with 10% PVA and 10% plasticizer

1.4 kg of HPS powder is mixed with 10 g of kappa-carrageenan and 175 g of PVA (PVA has a viscosity of 33 cps at 4% and 20°C). To 3.21 kg of deionised water under stirring is added 5 g of potassium acetate (0.10% by weight in the solution) and 175 g of glycerol (3.5% in solution and 10% in capsule), followed by addition of the above mixture (28% of HPS, 0.20% of carrageenan and 3.5% of PVA in solution). After the HPS is well dispersed, the dispersion is heated upto 90°C under slow stirring, then held under strong stirring for 30 minutes to assure a good solubilisation of the components.

The HPS solution thus prepared is defoamed under slow stirring and then poured into a dipping dish of a pilot machine of conventional hard gelatine capsule production equipment. While keeping the dipping HPS solution at 60°C, natural transparent hard capsules of size 0 were produced according to the conventional process with the same dimensional specifications to the conventional hard gelatine capsules.

### Disintegration test results:

| | |
|---|---|
| First leak time | 51 seconds |
| Total disintegration time | 225 seconds |

### Example 3: Production of HES capsules with 10% plasticizer

1.30 kg of HES powder is mixed with 4.00 g of gellan. To 3.55 kg of deionised water under stirring is added 5.00 g of potassium acetate (0.10% by weight in the solution), 2.00 g of ethylenediaminetetraacetic acid disodium salt (0.04%) and 145 g of glycerol (2.90% in solution and 10% in capsule), followed by addition of the above mixture (26.0% of HES and 0.08% of gellan in solution). After the HES is well dispersed, the dispersion is heated upto 98°C under slow stirring, then held under strong stirring for 10 minutes to assure a good solubilisation of the components.

The HES solution thus prepared is defoamed under slow stirring and then poured into a dipping dish of a pilot machine of conventional hard gelatine capsule production equipment. While keeping the dipping HES solution at 60°C, natural transparent hard capsules of size 0 were produced according to the conventional process with the same dimensional specifications to the conventional hard gelatine capsules.

### Disintegration test results:

| | |
|---|---|
| First leak time | 28 seconds |
| Total disintegration time | 443 seconds |

## Claims

1. An aqueous solution of a film forming composition for the manufacture of hard capsules, the composition consisting of:
hydroxypropylated starch or hydroxyethylated starch in an amount of 20 to 40 % by weight of the aqueous solution, and a setting system consisting of cations and a hydrocolloid or mixtures of hydrocolloids wherein hydrocolloids are present in an amount of 0.03 to 1.0 % by weight of the aqueous solution and wherein cations are present in an amount of 0.01 to 1 % by weight of the aqueous solution.

2. The aqueous solution according to claim 1, wherein the setting system contains optionally sequestering agents.

3. The aqueous solution according to claim 1, wherein the aqueous solution optionally contains sequestering agents in an amount of 0.001 to 5 %, preferably 0.01 to 3 % by weight of the aqueous solution.

4. The aqueous solution according to claim 1, wherein the hydrocolloids of the setting system are selected from polysaccharides.

5. The aqueous solution according to claim 1, wherein the hydrocolloids of the setting system are selected from alginates, agar gum, guar gum, locust bean gum (carob), carrageenan, tara gum, gum arabic, ghatti gum, Khaya grandifolia gum, tragacanth gum, karaya gum, pectin, arabian (araban), xanthan, gellan, starch, Konjac mannan, galactomannan, or funoran.

6. The aqueous solution according to claim 1, wherein the hydrocolloids of the setting system are selected from exocellular polysaccharides.

7. The aqueous solution according to claim 1, wherein the hydrocolloids of the setting system are selected from xanthan, acetan, gellan, welan, rhamsan, furcelleran, succinoglycan, scleroglycan, schizophyllan, tamarind gum, curdlan, pullulan, or dextran.

8. The aqueous solution according to claim 1, wherein the hydrocolloids of the setting system are selected from gellan gum or kappa-carrageenan.

9. The aqueous solution according to claim 1, wherein the optional sequestering agent or mixture of sequestering agents of the setting system is selected from ethylenediaminetetraacetic acid, acetic acid, boric acid, citric acid, edetic acid, gluconic acid, lactic acid, phosphoric acid, tartaric acid or salts thereof, methaphosphates, dihydroxyethylglycine, lecithin or beta cyclodextrin.

10. The aqueous solution according to claim 9, wherein the sequestering agent or mixture of sequestering agents is selected from ethylenediaminetetraacetic acid or salts thereof or citric acid or salts thereof.

11. The aqueous solution according to claims 1 containing additionally plasticizers in a range from 0 to 20 % based upon the weight of the composition.

12. The aqueous solution according to claim 11 wherein the plasticizer or mixture of plasticizers is selected from polyethylene glycol, glycerol, sorbitol, sucrose, corn syrup, fructose, dioctyl-sodium sulfosuccinate, triethyl citrate, tributyl citrate, 1,2-propylenglycol, mono-, di-or triacetates of glycerol, or natural gums.

13. The aqueous solution according to claim 1 containing additionally colouring agents in a range from 0 to 10 % based upon the weight of the composition.

14. The aqueous solution according to claim 13 wherein the colouring agent or mixture of colouring agents is selected from azo-, quinophthalone-, triphenylmethane-, xanthene-or indigoid dyes, iron oxides or hydroxides, titanium dioxide or natural dyes.

15. The aqueous solution according to claim 14 wherein the colouring agent or mixture of colouring agents is selected from patent blue V, acid brilliant green BS, red 2G, azorubine, ponceau 4R, amaranth, D+C red 33, D+C red 22, D+C red 26, D+C red 28, D+C yellow 10, yellow 2 G, FD+C yellow 5, FD+C yellow 6, FD+C red 3, FD+C red 40, FD+C blue 1, FD+C blue 2, FD+C green 3, or brilliant black BN.

16. The aqueous solution according to claim 13 wherein the colouring agent or mixture of colouring agents is selected from carbon black, iron oxide black, iron oxide red, iron oxide yellow, titanium dioxide, riboflavin, carotenes, anthocyanines, turmeric, cochineal extract, clorophyllin, canthaxanthin, caramel, or betanin.

17. A pharmaceutical hard capsule for unit dosage forms for agrochemicals, seeds, herbs, foodstuffs, dyestuffs, pharmaceuticals, or flavouring agents produced from the aqueous solution according to claims 1 to 16.

18. The capsule according to claim 17, **characterized in that** it has a coating.

19. The capsule according to claim 18, wherein the coating is selected from cellulose acetate phthalate, polyvinyl acetate phthalate, methacrylic acid gelatines, hypromellose phthalate, hydroxypropylmethyl cellulose phthalate hydroxyalkyl methyl cellulose phthalates or mixtures thereof.

20. Caplets encapsulated in an aqueous solution according to claim 1.

21. The capsule according to claim 17 **characterized in that** the capsule halves are sealed with one or more layers of the aqueous solution according to claims 1 to 16.

22. The capsule according to claim 17 **characterized in that** a liquid fusion process seals the capsule halves.

23. The capsule according to claim 17 **characterized by** a release of filled product at low temperature, such as at room temperature.

24. Use of aqueous solutions according to claim 1 for the manufacturing of hard capsules in a dip moulding process.

25. Manufacturing of hard capsules from a hydroxypropylated starch aqueous solution according to claims 1 to 16, in a dip moulding process with conventional hard gelatine capsules process parameters and equipment.

## Patentansprüche

1. Wässrige Lösung einer filmbildenden Zusammensetzung für die Herstellung von harten Kapseln, wobei die Zusammensetzung aus:
hydroxypropylierter Stärke oder hydroxyethylierter Stärke in einer Menge von 20 bis 40 Gew.-% der wässrigen Lösung und einem Härtungssystem, das aus Kationen und einem Hydrokolloid oder Hydrokolloidgemischen besteht, besteht, wobei die Hydrokolloide in einer Menge von 0,03 bis 1,0 Gew.-% der wässrigen Lösung vorliegen und wobei die Kationen in einer Menge von 0,01 bis 1 Gew.-% der wässrigen Lösung vorliegen.

2. Wässrige Lösung nach Anspruch 1, wobei das Härtungssystem gegebenenfalls Sequestriermittel enthält.

3. Wässrige Lösung nach Anspruch 1, wobei die wässrige Lösung gegebenenfalls Sequestriermittel in einer Menge von 0,001 bis 5 Gew.-%, vorzugsweise von 0,01 bis 3 Gew.-%, der wässrigen Lösung enthält.

4. Wässrige Lösung nach Anspruch 1, wobei die Hydrokolloide des Härtungssystems aus Polysacchariden ausgewählt sind.

5. Wässrige Lösung nach Anspruch 1, wobei die Hydrokolloide des Härtungssystems aus Alginaten, Agargummi, Guargummi, Johannisbrotgummi (Carob), Carrageenan, Taragummi, Gummi arabicum, Ghattigummi, Khaya-grandifolia-Gummi, Tragantgummi, Karayagummi, Pektin, Arabinan (Araban), Xanthan, Gellan, Stärke, Konjac-Mannan, Galactomannan und Funoran ausgewählt sind.

6. Wässrige Lösung nach Anspruch 1, wobei die Hydrokolloide des Härtungssystems aus exozellulären Polysacchariden ausgewählt sind.

7. Wässrige Lösung nach Anspruch 1, wobei die Hydrokolloide des Härtungssystems aus Xanthan, Acetan, Gellan, Welan, Rhamsan, Furcelleran, Succinoglycan, Scleroglycan, Schizophyllan, Tamarindengummi, Curdlan, Pullulan und Dextran ausgewählt sind.

8. Wässrige Lösung nach Anspruch 1, wobei die Hydrokolloide des Härtungssystems aus Gellangummi und kappa-Carrageenan ausgewählt sind.

9. Wässrige Lösung nach Anspruch 1, wobei das optionale Sequestriermittel oder das optionale Gemisch von Sequestriermitteln des Härtungssystems aus Ethylendiamintetraessigsäure, Essigsäure, Borsäure, Citronensäure, Edetinsäure, Gluconsäure, Milchsäure, Phosphorsäure, Weinsäure und Salzen derselben, Metaphosphaten, Dihydroxyethylglycin, Lecithin und beta-Cyclodextrin ausgewählt ist.

10. Wässrige Lösung nach Anspruch 9, wobei das Sequestriermittel oder das Gemisch von Sequestriermitteln aus Ethylendiamintetraessigsäure und Salzen davon und Citronensäure und Salzen davon ausgewählt ist.

11. Wässrige Lösung nach Anspruch 1, enthaltend zusätzlich Weichmacher in einem Bereich von 0 bis 20%, bezogen auf das Gewicht der Zusammensetzung.

12. Wässrige Lösung nach Anspruch 11, wobei der Weichmacher oder ein Gemisch von Weichmachern aus Polyethylenglykol, Glycerin, Sorbit, Saccharose, Maisstärkesirup, Fructose, Dioctylnatriumsulfosuccinat, Triethylcitrat, Tributylcitrat, 1,2-Propylenglykol, Mono-, Di- oder Triacetaten von Glycerin und natürlichen Gummen ausgewählt ist.

13. Wässrige Lösung nach Anspruch 1, die zusätzlich Färbemittel in einem Bereich von 0 bis 10%, bezogen auf das Gewicht der Zusammensetzung, enthält.

14. Wässrige Lösung nach Anspruch 13, wobei das Färbemittel oder ein Gemisch von Färbemitteln aus Azo-, Chinophthalon-, Triphenylmethan-, Xanthen- oder Indigofarbstoffen, Eisenoxiden oder -hydroxiden, Titandioxid und natürlichen Farbstoffen ausgewählt ist.

15. Wässrige Lösung nach Anspruch 14, wobei das Färbemittel oder das Gemisch von Färbemitteln aus Patent Blue V, Acid Brillian Green BS, Red 2G, Azorubin, Ponceau 4R, Amaranth, D+C Red 33, D+C Red 22, D+C Red 26, D+C Red 28, D+C Yellow 10, Yellow 2 G, FD+C Yellow 5, FD+C Yellow 6, FD+C Red 3, FD+C Red 40, FD+C Blue 1, FD+C Blue 2, FD+C Green 3 oder Brilliant Black BN ausgewählt ist.

16. Wässrige Lösung nach Anspruch 13, wobei das Färbemittel oder das Gemisch von Färbemittels aus Kohleschwarz, Eisenoxidschwarz, Eisenoxidrot, Eisenoxidgelb, Titandioxid, Riboflavin, Carotinen, Anthocyaninen, Kurkuma, Cochenille-Extrakt, Chlorophyllin, Canthaxanthin, Karamell oder Betanin ausgewählt ist.

17. Pharmazeutische harte Kapsel für Einheitsdosisformen für Agrochemikalien, Saatgut, Kräuter, Nahrungsmittel, Farbstoffe, Arzneimittel oder Aromastoffe, die aus der wässrigen Lösung nach den Ansprüchen 1 bis 16 hergestellt wurde.

18. Kapsel nach Anspruch 17, **dadurch gekennzeichnet, dass** sie einen Überzug aufweist.

19. Kapsel nach Anspruch 18, wobei der Überzug aus Celluloseacetatphthalat, Polyvinylacetatphthalat, Methacrylsäure-Gelatinearten, Hypromellosephthalat, Hydroxypropylmethylcellulosephthalat, Hydroxyalkylmethylcellulosephthalaten und Gemischen davon ausgewählt ist.

20. Kapseltabletten (Caplets), die in eine wässrige Lösung nach Anspruch 1 eingekapselt sind.

21. Kapsel nach Anspruch 17, **dadurch gekennzeichnet , dass** die Kapselhälften mit einer Schicht oder mehreren Schichten der wässrigen Lösung nach den Ansprüchen 1 bis 16 versiegelt sind.

22. Kapsel nach Anspruch 17, **dadurch gekennzeichnet, dass** ein Flüssigschmelzverfahren die Kapselhälften versiegelt.

23. Kapsel nach Anspruch 17, **gekennzeichnet durch** eine Freisetzung des Füllproduktes bei niedriger Temperatur, zum Beispiel bei Raumtemperatur.

24. Verwendung von wässrigen Lösungen nach Anspruch 1 zur Herstellung von harten Kapseln in einem Tauchformverfahren.

25. Herstellung von harten Kapseln aus einer wässrigen Lösung von hydroxypropylierter Stärke nach den Ansprüchen 1 bis 16 in einem Tauchformverfahren mit herkömmlichen Verfahrensparametern und -anlagen für Hartgelatinekapseln.

## Revendications

1. Solution aqueuse d'une composition filmogène pour la fabrication de gélules dures, la composition consistant en :
amidon hydroxypropylé ou d'amidon hydroxyéthylé en une quantité allant de 20 à 40 % en poids de la solution aqueuse, et en un système de prise en masse consistant en cations et d'un hydrocolloïde ou de mélanges d'hydrocolloïdes, les hydrocolloïdes étant présents en une quantité allant de 0,03 à 1,0 % en poids de la solution aqueuse et les cations étant présents en une quantité allant de 0,01 à 1 % en poids de la solution aqueuse.

2. Solution aqueuse selon la revendication 1, dans laquelle le système de prise en masse contient facultativement des agents séquestrants.

3. Solution aqueuse selon la revendication 1, la solution aqueuse contenant facultativement des agents séquestrants en une quantité allant de 0,001 à 5 %, de préférence de 0,01 à 3 %, en poids de la solution aqueuse.

4. Solution aqueuse selon la revendication 1, dans laquelle les hydrocolloïdes du système de prise en masse sont sélectionnés parmi les polysaccharides.

5. Solution aqueuse selon la revendication 1, dans laquelle les hydrocolloïdes du système de prise en masse sont sélectionnés parmi les alginates, la gomme d'agar, la gomme de guar, la gomme de fève de caroubier (caroube), la carragénine, la gomme de tara, la gomme arabique, la gomme de ghatti, la gomme de Khaya grandifolia, la gomme adragante, la gomme de karaya, la pectine, la gomme arabique (araban), le xanthane, le gellane, l'amidon, le mannane de konjac, le galactomannane et le funoran.

6. Solution aqueuse selon la revendication 1, dans laquelle les hydrocolloïdes du système de prise en masse sont sélectionnés parmi les polysaccharides exocellulaires.

7. Solution aqueuse selon la revendication 1, dans laquelle les hydrocolloïdes du système de prise en masse sont sélectionnés parmi le xanthane, l'acétane, le gellane, le wellane, le rhamsane, le furcellarane, le succinoglycane, le scléroglucane, le schizophyllane, la gomme de tamarin, le curdlane, le pullulane et le dextrane.

8. Solution aqueuse selon la revendication 1, dans laquelle les hydrocolloïdes du système de prise en masse sont sélectionnés parmi la gomme gellane et le kappa-carraghénane.

9. Solution aqueuse selon la revendication 1, dans laquelle l'agent séquestrant, ou le mélange d'agents séquestrants, facultatif du système de prise en masse est sélectionné parmi l'acide éthylènediamine-tétraacétique, l'acide acétique, l'acide borique, l'acide citrique, l'acide édétique, l'acide gluconique, l'acide lactique, l'acide phosphorique, l'acide tartrique ou leurs sels, les métaphosphates, la dihydroxyéthylglycine, la lécithine et la bêta-cyclodextrine.

10. Solution aqueuse selon la revendication 9, dans laquelle l'agent séquestrant ou le mélange d'agents séquestrants est sélectionné parmi l'acide éthylènediaminetétraacétique ou ses sels, et l'acide citrique ou ses sels.

11. Solution aqueuse selon la revendication 1, contenant en outre des agents plastifiants à raison d'environ 0 à 20 % exprimés par rapport au poids de la composition.

12. Solution aqueuse selon la revendication 11, dans laquelle l'agent plastifiant ou le mélange d'agents plastifiants est sélectionné parmi le polyéthylène glycol, le glycérol, le sorbitol, le saccharose, le sirop de maïs, le fructose, le dioctyl-sulfosuccinate de sodium, le citrate de triéthyle, le citrate de tributyle, le 1,2-propylène glycol, les mono-, di- ou triacétates de glycérol, et les gommes naturelles.

13. Solution aqueuse selon la revendication 1, contenant en outre des agents colorants à raison d'environ 0 à 10 % exprimés par rapport au poids de la composition.

14. Solution aqueuse selon la revendication 13, dans laquelle l'agent colorant ou le mélange d'agents colorants est sélectionné parmi les colorants azoïques, quinophtalone, triphénylméthane, xanthène ou indigoïdes, les oxydes ou hydroxydes de fer, le dioxyde de titane et des colorants naturels.

15. Solution aqueuse selon la revendication 14, dans laquelle l'agent colorant ou le mélange d'agents colorants est sélectionné parmi le bleu patenté V, le vert brillant acide BS, le rouge 2G, l'azorubine, le ponceau 4R, l'amaranthe, le rouge D+C 33, le rouge D+C 22, le rouge D+C 26, le rouge D+C 28, le jaune D+C 10, le jaune 2G, le jaune FD+C 5, le jaune FD+C 6, le rouge FD+C 3, le rouge FD+C 40, le bleu FD+C 1, le bleu FD+C 2, le vert FD+C 3 et le noir brillant BN.

16. Solution aqueuse selon la revendication 13, dans laquelle l'agent colorant ou le mélange d'agents colorants est sélectionné parmi le noir de carbone, le noir d'oxyde de fer, le rouge d'oxyde de fer, le jaune d'oxyde de fer, le dioxyde de titane, la riboflavine, les carotènes, les anthocyanines, la curcumine, l'extrait de cochenille, la chlorophylline, la canthaxanthine, le caramel et la bétanine.

17. Gélule pharmaceutique dure pour formes dosées unitaires de produits agrochimiques, graines, herbes, produits alimentaires, colorants, produits pharmaceutiques, ou agents aromatisants, obtenus à partir de la solution aqueuse selon les revendications 1 à 16.

18. Gélule selon la revendication 17, **caractérisé par le fait qu'**elle a un enrobage.

19. Gélule selon la revendication 18, dans laquelle l'enrobage est sélectionné parmi le phtalate d'acétate de cellulose, le poly(phtalate d'acétate de vinyle), les acide méthacrylique-gélatines, le phtalate d'hypromellose, le phtalate d'hydroxypropylméthylcellulose, les phtalates d'hydroxyalkylméthylcellulose, et leurs mélanges.

20. Caplets encapsulés dans une solution aqueuse selon la revendication 1.

21. Gélule selon la revendication 17, **caractérisée par le fait que** les demi-gélules sont scellées au moyen d'une ou plusieurs couches de la solution aqueuse selon les revendications 1 à 16.

22. Gélule selon la revendication 17, **caractérisée par le fait qu'**une opération de fusion liquide scelle les demi-gélules.

23. Gélule selon la revendication 17, **caractérisée par** la libération à basse température, telle qu'à température ambiante, du produit qu'elle renferme.

24. Utilisation de solutions aqueuses selon la revendication 1, pour la fabrication de gélules dures par un procédé de moulage par trempage.

25. Fabrication de gélules dures à partir d'une solution aqueuse d'amidon hydroxypropylé selon les revendications 1 à 16 par un procédé de moulage par trempage utilisant des paramètres de procédé et un équipement pour gélules de gélatine dure classiques.
